# EUROPEAN PATENT SPECIFICATION

(11) **EP 4 679 364 B1**
(45) Date of publication and mention of the grant of the patent: **26.08.2026**
(21) Application number: 25187994.6
(22) Date of filing: 08.07.2025
(51) Int. Cl.: G06T 7/00, A61B 34/10, A61B 18/14

(54) **METHODS AND SYSTEMS FOR IDENTIFYING CONDUCTION FLOW PATHS IN ANATOMICAL MAPS**
VERFAHREN UND SYSTEME ZUR IDENTIFIZIERUNG VON LEITUNGSSTRÖMUNGSWEGEN IN ANATOMISCHEN KARTEN
PROCÉDÉS ET SYSTÈMES D'IDENTIFICATION DE CHEMINS D'ÉCOULEMENT DE CONDUCTION DANS DES CARTES ANATOMIQUES

(30) Priority: 09.07.2024 US 202418766855
(43) Date of publication of application: 14.01.2026
(73) Proprietor: Biosense Webster (Israel) Ltd., 2066717 Yokneam (IL)
(72) Inventor: SEGEV, Meytal, 2066717 Yokneam (IL); ZAR, Lior, 2066717 Yokneam (IL); REUVENY, Hadar, 2066717 Yokneam (IL); YANOVICH, Nir, 2066717 Yokneam (IL); OSTROV, Itay, 2066717 Yokneam (IL)
(74) Representative: Carpmaels & Ransford LLP

(56) References cited:
- US-A1- 2017 265 926
- US-A1- 2021 169 365
- US-A1- 2024 203 035

## Description

### FIELD OF THE DISCLOSURE

This disclosure relates generally to cardiac ablation, and specifically to a system and method for real-time planning and monitoring of cardiac ablation using an anatomical map.

### BACKGROUND OF THE DISCLOSURE

Providing indications of ablation gaps on an anatomical map of an inner wall of a cardiac chamber was previously proposed in the patent literature. For example, U.S. patent 9,757,182 describes a method including receiving locations of multiple ablation sites formed on the surface of a heart. Distances are measured among at least some of the ablation sites based on the locations. One or more gaps between the ablation sites, which meet an alerting criterion, are identified. The identified gaps are indicated to an operator.

Other relevant prior art documents include:
US 2021/169365 Al relating to systems and methods for ablating cardiac tissue by determining cardiac targets;
US2024/203035 A1 relating to systems and methods for using signed distance functions to visualize pulsed field ablation (PFA) tags.

US2017/265926 relating to methods and systems of identification and visualization of gaps between cardiac ablation sites.

The present disclosure will be more fully understood from the following detailed description of the examples thereof, taken together with the drawings, in which:

### BRIEF DESCRIPTION OF THE DRAWINGS

Fig. 1 is a schematic, pictorial illustration of a catheter-based electroanatomical (EA) mapping and ablation system, in accordance with an example of the present disclosure;
Figs. 2A and 2B are anatomical maps of cardiac wall tissue schematically superimposed with (a) ablation tags and derived non conducting zones, and (b) simulated
   conduction paths, respectively, in accordance with an example of the present disclosure;
Fig. 3 is a flow chart that schematically illustrates a method for deriving and displaying possible remaining conduction flow paths over an anatomical map, in accordance with an example of the present disclosure; and
Fig. 4 is a schematic illustration of a graphical user interface (GUI) used for selecting preferences in modeling possible remaining conduction flows, in accordance with an example of the present disclosure.

### DETAILED DESCRIPTION OF EXAMPLES

### OVERVIEW

In a catheter-based cardiac ablation procedure devised to treat an arrhythmia, such as pulmonary vein isolation (PVI) atrial fibrillation (AFib), a physician ablates tissue in a specific anatomical region, (e.g., over an entire circumference of an ostium of a PV). The ablation, such as one using a pulsed-field ablation (PFA) technique, may require several iterations to cover the entire circumference of the ostium. Each iteration requires moving an ablation catheter to an area where the arrhythmogenic conduction has not yet been blocked by ablation.

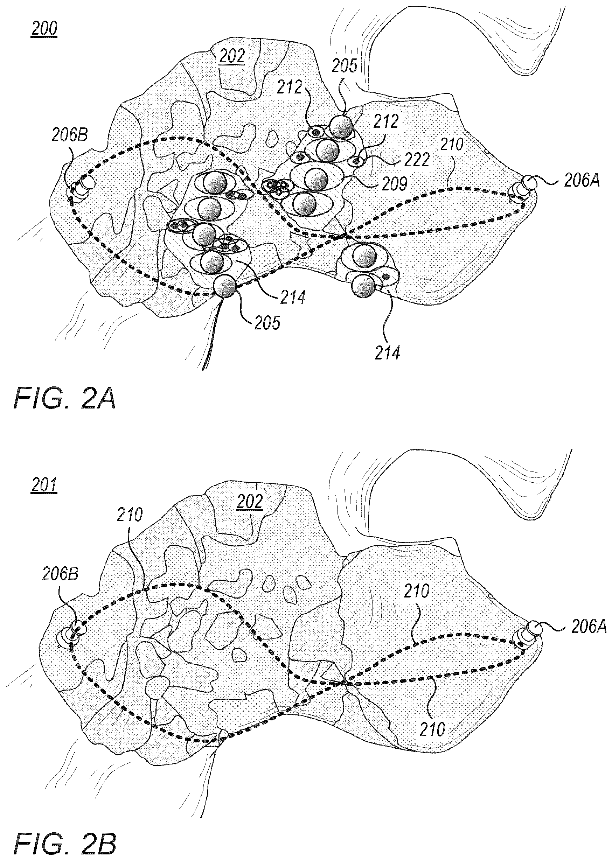

To successfully complete the ablation, the physician may be assisted by ablation tags distributed over a surface of a 3D anatomical map (seen schematically in Fig. 2A) that identify clinically relevant surface tissue areas that have not yet been ablated.

Several ablation tag types may be used, such as (i) small but densely spread tags (applied over a grid of ablation points) to delineate tissue areas that received diffused ablative power, and (ii) larger and more dispersed tags that designate point locations where a catheter applied ablation.

However, a very large number of ablation tags superimposed over the map surface can cause visual clutter. Moreover, it is still difficult to make a conclusive determination about any area displaying a mixture of ablation tags that indicate partial (e.g., incomplete) ablation along with tags that indicate full ablation.

The above-described challenges due to the visual noise caused by the tags can have a negative impact on the physician's ability to (i) align a visualized 3D location of the catheter with a previous ablation location, (ii) evaluate the capacity of tissue damage created, and (iii) identify gaps in ablation.

Some examples of the present disclosure described herein provide a real-time visualization technique to identify possible remaining conduction flow paths on a map (as seen in Fig. 2B) that can aid a physician to cope with the challenges described above. The technique uses ablation tag information to calculate possible paths, via candidate breakthrough areas, that are left after a prior ablation. The visualized paths focus the physician's consideration on whether, and where, further ablations may be required to eliminate an arrhythmia, and, at the same time, hide the ablation tags to eliminate visual clutter.

The possible remaining paths are calculated using a "first search" algorithm, such as the A* algorithm, one of many first search algorithm types that are widely discussed in scientific and engineering literature. One example is a paper entitled "Generalized Best First Search Strategies and the Optimality of A*," by R. Dechter and J. Pearl, published in the Journal of the Association for Computing Machinery, Volume 32, No. 3, pp. 505-536 (1985).

Other pathfinding algorithms may be used, such as the D* algorithm, which is also suitable for use in real time. The D* algorithm can further assist the physician in finalizing ablation as the physician progressively blocks (e.g., ablates) residual conduction paths. A paper describing the D* algorithm titled, "The Focused D* Algorithm for Real-Time Re-planning" was published by A. Stentz in Proceedings of the International Joint Conference on Artificial Intelligence: pages 1652-1659, (1995).

In an example of the disclosed technique, a processor runs an algorithm that performs the following steps:
1. Defining a scar region (e.g., a sphere of a given radius) around each ablation tag for at least some of the ablation tags. The size of the scar region (e.g., sphere's radius) can be customized by the algorithm according to the level of ablation (e.g., using an existing ablation index scale or touch proximity index (TPI)) to reflect high-certainty block areas and possible conductive areas in addition to the gaps, as seen in Fig. 2A. In another example, the user can also configure the radius based on another consideration (e.g., experience), with no relation to ablation index or TPI.
   The A* algorithm uses the defined scar regions as being "Blocked" for a possible path as the first spatial input.
2. Letting the user indicate, on the map, a start point and an ending point of possible paths on both sides of an ablation line of candidate breakthrough areas, as seen in Fig. 2A, to query if an conduction flow through a candidate breakthrough area can occur.
   The A* algorithm uses the user's starting point (source) location on map and ending point (target) location on the map as the second spatial input to define the directionality of the possible paths.
3. Running the first search algorithm (e.g., A* algorithm) to calculate possible existing paths and optionally indicate possible conduction breakage zones within a map area of interest.
   The A* algorithm uses the first and second spatial inputs in computing all possible paths between the marked source and the marked target considering the "Blocked" regions.
4. Visualizing the calculated paths on the map to show the user only possible remaining paths of the flow of conduction, as seen in Fig. 2B. The ablation tags are then hidden to reduce visual clutter.

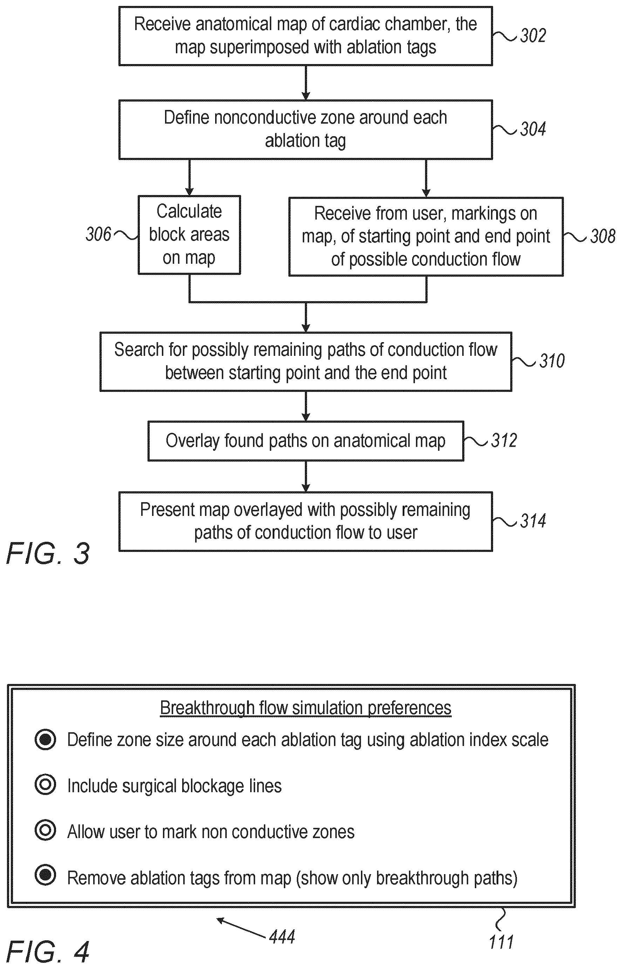

In an example, shown in Fig. 4, a graphical user interface (GUI) provided by the disclosed technique allows a user to decide whether or not the algorithm should consider the level of ablation at each tag location. The exemplified GUI also allows a user to include block areas created during a past surgical procedure. The user may further decide if the ablation tags over the map, now overlayed with the paths, will remain visible.

The displayed paths of possible remaining conduction flow on the map may ease the work of a physician because (a) only part of the map must be checked, and (b) clearer guidance about ambiguous map regions is received. Note, though the presented paths can help optimize workflow (e.g., to focus attention and ease the workload of the physician), they are not intended as a proposal for any specific ablation locations. Rather, such later clinical steps are left to the judgment of the physician.

Finally, while the disclosed technique is demonstrated in relation to the ablation treatment of AFib, it is applicable to ablation treatments of other types of arrhythmias, such as ventricular arrhythmias.

### SYSTEM DESCRIPTION

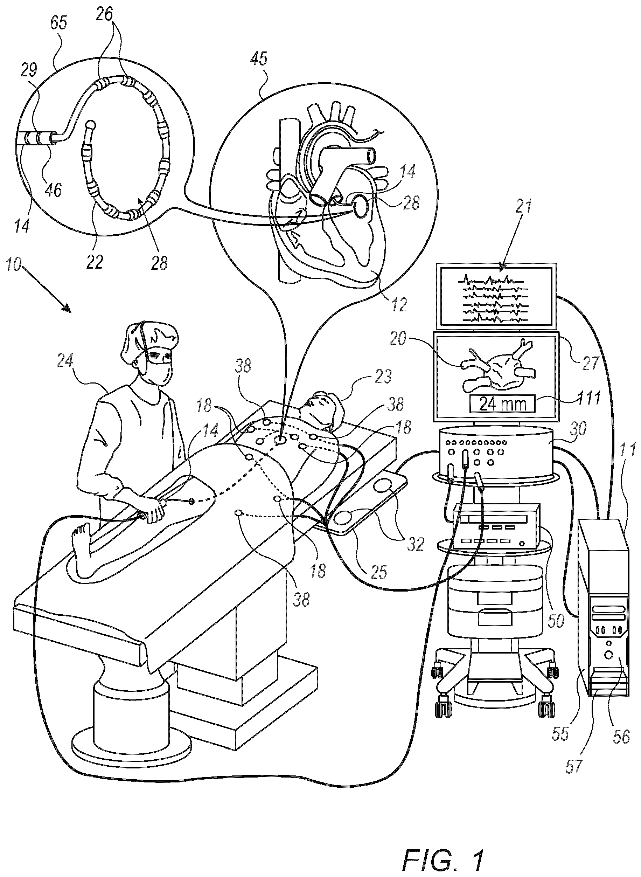

Fig. 1 is a schematic, pictorial illustration of a catheter-based electroanatomical (EA) mapping and ablation system 10, in accordance with an example of the present disclosure.

System 10 includes multiple catheters, which are percutaneously inserted by physician 24 through the patient's vascular system into a chamber or vascular structure of a heart 12 (seen in inset 45). Typically, a delivery sheath catheter is inserted into a cardiac chamber, such as the left or right atrium near a desired location in heart 12. Thereafter, a plurality of catheters can be inserted into the delivery sheath catheter to arrive at the desired location. The plurality of catheters may include a catheter dedicated to pacing, a catheter for sensing intracardiac electrogram signals, a catheter dedicated to ablating and/or a catheter dedicated to both EA mapping and ablating. An example catheter 14, illustrated herein, is configured for sensing bipolar electrograms and pulsed field ablation (PFA). Physician 24 brings a distal tip 28 (also called hereinafter distal end assembly 28) of catheter 14 into contact with the heart wall for ablating a target site in heart 12.

As seen in inset 65, catheter 14 is an exemplary catheter that includes a lasso distal end assembly 28, including one, and preferably multiple, electrodes 26 optionally distributed over a curved spline 22. Catheter 14 may additionally include a position sensor 29, embedded in or near distal tip 28 on a shaft 46 of catheter 14, to track the position and orientation of distal tip 28. Optionally, and preferably, position sensor 29 is a magnetic-based position sensor including three magnetic coils for sensing three-dimensional (3D) position and orientation.

Magnetic-based position sensor 29 may be operated together with a location pad 25 that includes a plurality of magnetic coils 32 configured to generate magnetic fields in a predefined working volume. The real-time position of distal tip 28 of catheter 14 may be tracked based on magnetic fields generated with location pad 25 and sensed by magnetic-based position sensor 29. Details of the magnetic-based position sensing technology are described in U.S. Patent Nos. 5,5391,199; 5,443,489; 5,558,091; 6,172,499; 6,239,724; 6,332,089; 6,484,118; 6,618,612; 6,690,963; 6,788,967; 6,892,091.

System 10 includes one or more electrode patches 38 positioned for skin contact on patient 23 to establish a location reference for location pad 25 as well as impedance-based tracking of electrodes 26. For impedance-based tracking, electrical current is directed toward electrodes 26 and sensed at electrode skin patches 38 so that the location of each electrode can be triangulated via electrode patches 38. Details of the impedance-based location tracking technology are described in US Patent Nos. 7,536,218; 7,756,576; 7,848,787; 7,869,865; and 8,456,182.

A recorder 11 displays cardiac signals 21 (e.g., electrograms acquired at respectively tracked cardiac tissue positions) acquired with body surface ECG electrodes 18 and intracardiac electrograms acquired with electrodes 26 of catheter 14. Recorder 11 may include pacing capability to pace the heart rhythm, and/or may be electrically connected to a standalone pacer.

System 10 may include an ablation energy generator 50 adapted to conduct ablative energy to one or more electrodes at a distal tip of a catheter configured for ablation. The energy produced by ablation energy generator 50 may include, but is not limited to, radiofrequency (RF) energy or pulse field (PF) energy, including monopolar or bipolar and monophasic of biphasic high-voltage DC pulses, to be used to effect irreversible electroporation (IRE) or combinations thereof.

The patient interface unit (PIU) 30 is an interface configured to establish electrical communication between catheters, electrophysiological equipment, power supply, and a workstation 55 to control system 10 operation and receive EA signals from the catheter. Electrophysiological equipment of system 10 may include, for example, multiple catheters, location pad 25, body surface ECG electrodes 18, electrode patches 38, ablation energy generator 50, and recorder 11. Optionally, and preferably, PIU 30 additionally includes processing capability for implementing real-time computations of catheter locations and for performing ECG calculations.

Workstation 55 includes memory 57, a processor 56 unit with memory or storage with appropriate operating software loaded therein, and user interface capability. Workstation 55 may provide multiple functions, optionally including (i) modeling endocardial anatomy in three-dimensions (3D) and rendering the model or anatomical map 20 for display on a display device 27, (ii) displaying on display device 27 activation sequences (or other data) compiled from recorded cardiac signals 21 in representative visual indicia or imagery superimposed on the rendered anatomical map 20, (iii) displaying real-time location and orientation of multiple catheters within the heart chamber, and (iv) displaying sites of interest on display device 27 such as places where ablation energy has been applied. One commercial product embodying elements of system 10 is available as the CARTO^{™} 3 System, available from Biosense Webster, Inc., 31A Technology Drive, Irvine, CA 92618.

In the disclosed example, processor 56 runs an algorithm that defines a nonconductive zone around each ablation tag for at least some of the ablation tags on an anatomical map, such as map 20. Physician 24 marks a start point and an ending point of a potential conduction wave flow on the map, and processor 56 then applies a first search algorithm to simulate a conduction wave flow from the start point to the ending point. The processor overlays any found conduction flow path on the anatomical map and presents the resulting map (e.g., such as seen in Fig. 2B) to the physician.

In some examples, processor 56 typically comprises a general-purpose computer programmed in software to carry out the functions described herein. The software may be downloaded to the computer in electronic form over a network, for example, or it may, alternatively or additionally, be provided and/or stored on non-transitory tangible media, such as magnetic, optical, or electronic memory.

This configuration of system 10 is shown by way of example, to illustrate certain problems that are addressed by examples of the present disclosure and to demonstrate the application of these examples in enhancing the performance of such a system. Examples of the present disclosure, however, are by no means limited to this specific sort of example system, and the principles described herein may similarly be applied to other sorts of medical systems. For example, other multi-electrode catheter types may be used, such as a basket catheter.

### ARRHYTHMIA BREAKTHROUGH FLOW SIMULATION

Figs. 2A and 2B are anatomical maps 200 and 201 of cardiac wall tissue that are schematically superimposed with (a) ablation tags (205, 212) and derived non-conducting zones 214, and (b) simulated conduction paths 210, respectively, in accordance with an example of the present disclosure.

Ablation tags 205 designate point locations where a catheter applied ablation. Ablation tags 212 delineate ablated tissue areas (e.g., tags 212 trace surface tissue locations that received diffused ablation power). The small but densely spread tags 212 are applied over a grid of ablation points that cover ablated areas. Larger and more dispersed tags 205 are applied subsequent to ablation events.

A processor that runs the disclosed algorithm defines a scar region (e.g., a sphere 209 or 222 of given radiuses, depending on ablation index or custom user configuration unrelated to any of the ablation parameters) about each respective ablation tag 205 or 212 for at least some of the ablation tags. Each sphere's intersection with the map's surface defines an area. Each intersection area or a union of these intersection areas defines block areas 214. The user can customize the scar tag configuration (e.g., radius size), according to the ablation index scale, to reflect high certainty block areas 214, as seen in Fig. 2A.

The user marks a start point 206A and an ending point 206B on map 200 to query if any potential conduction paths between the points are left. The start and ending points are located on both sides of an ablation line, marking an area of interest within which the user wishes to determine potential remaining conduction.

The processor searches (e.g., simulates using the A* algorithm) for the existence of possible conduction paths from start point 206A to ending point 206B. Any possible paths 210 of conduction flow found are overlayed on anatomical map 200. The ablation tags are then hidden to avoid visual clutter, as seen in map 201 of Fig. 2B.

The A* algorithm uses the defined scar regions (209, 222) as being "Blocked" (214) for a possible path (210) as the first spatial input. The A* algorithm uses the user's starting point (source) location on map 200 and ending point (target) location 200 on the map as the second spatial input to define the directionality of the possible paths. The A* algorithm uses the first and second spatial inputs in computing all possible paths between the marked source and the marked target considering the "Blocked" regions.

Map 201 typically lets the physician investigate a possible path 210 of a remaining conduction flow using, for example, electrophysiology analysis tools to determine a specific location on a path worth ablating. To this end map 200 may be an EA map, such as a late activation (LAT) map.

Figures 2A and 2B are brought by way of example. The user can, as an example, select different start points and ending points on the map to query other possible routes of conduction waves.

### METHOD OF DERIVING AND DISPLAYING POSSIBLE REMAINING CONDUCTION FLOW PATHS

Fig. 3 is a flow chart that schematically illustrates a method for deriving and displaying possible remaining conduction flow paths 210 over an anatomical map 200, in accordance with an example of the present disclosure. The algorithm, according to the presented example, carries out a process that begins with processor 56 receiving anatomical map 200 superimposed with ablation tags (205, 212), at an ablation map receiving step 302.

Next, processor 56 defines a sphere of nonconductive zone (e.g., sphere) around each ablation tag for at least some of the ablation tags, at non-conducting zones definition step 304.

Next, the processor calculates block areas 214, as described in Fig. 2A, at block areas calculation step 306.

At user query step 308, the processer receives from the user, markings of a start point and an ending point on map 200 to query for any possible remaining conduction flow paths between the points. For example, the user clicks both sides of an ablation line created by block areas 214, as seen in Fig. 2A, within which the user wishes to determine candidate remaining paths of conduction.

Using block areas 214 and the user query as inputs, the processor runs an algorithm (e.g., A* algorithm) that searches for paths between the start point to the ending point, at path simulation step 310.

At an overlaying step 312, the processor overlays any found possible remaining paths 210 of conduction flow on anatomical map 200, resulting in map 201 of Fig. 2B. The ablation tags can be presented or not on map 201 based on visualization setup configuration (e.g., using GUI 111 as described Fig.3). The block areas that the algorithm defined are not presented/visualized on map 201.

Finally, at map presentation step 314, the processor presents the overlayed map 201 on a display to a user.

### PREFERENCES GUI FOR DERIVING AND DISPLAYING POSSIBLE REMAINING CONDUCTION FLOW PATHS

Fig. 4 is a schematic illustration of a graphical user interface (GUI) 111 used for selecting preferences (444) to model possible remaining conduction flows, in accordance with an example of the present disclosure.

A user of GUI 111 can select (444) to define the sphere sizes around the scar zones, according to his professional judgment, by marking one of the checkboxes on the GUI. Selecting another checkbox displays the resulting map without the ablation tags (presenting only any possible remaining paths), as shown in Fig. 2B.

The user may choose to include surgically induced blockage areas, if documented.

Finally, the user may choose to mark (e.g., using a computer mouse) an area the physician deems irrelevant within which to search for a path.

The GUI of Fig. 4 is brought by way of example, where different GUI alternatives can be included in the disclosed technique. For example, a GUI that includes a menu to control graphical encoding of any found path can be provided. Another GUI may allow a user to select and mark multiple pairs of start and ending points between which to search for paths, and, according to their different start and ending points, to encode any found paths in a graphically different manner.

Although the examples described herein mainly address cardiac diagnostic applications, the methods and systems described herein can also be used in other medical applications.

## Claims

1. A method, comprising:
receiving an anatomical map (200) of wall tissue of at least a portion of a cardiac chamber, the map superimposed with ablation tags (205, 212);
defining a nonconductive zone (209) around each ablation tag (205, 212) for at least some of the ablation tags, to calculate block areas (214);
receiving from a user, markings on the map (200) of a starting point (206A) and an ending point (206B) of a potential conduction flow;
using a search algorithm, searching for one or more possible conduction flow paths (210) from the starting point (206A) to the ending point (206B), the one or more existing paths (210) taking into consideration the block areas (214);
overlaying one or more flow paths (210), found by the search algorithm, on the anatomical map; and
presenting the overlayed anatomical map (201) to a user.

2. The method according to claim 1, and comprising not displaying the ablation tags (205, 212) on the overlayed anatomical map (201).

3. The method according to claim 1, wherein the nonconductive zone (209) around a given ablation tag (205, 212) is a sphere of a given size derived from an ablation index value of the given ablation tag.

4. The method according to claim 3, wherein calculating the block areas (214) comprises calculating an intersection of the sphere and a surface of the anatomical map (201).

5. The method according to claim 1, wherein searching for the one or more conduction flow paths (210) comprises using a first search algorithm.

6. The method according to claim 5, wherein using a first search algorithm comprises using an A* path search algorithm.

7. The method according to claim 1, and comprising providing a graphical user interface (GUI) configured to allow the user to select at least one of (i) whether to show the ablation tags (205, 212) on the overlayed anatomical map (201), and (ii) whether to calculate sizes of nonconductive zones (209) according to a level of existing ablation therein.

8. A system, comprising:
an interface (30) configured to receive an anatomical map (200) of wall tissue of at least a portion of a cardiac chamber, the map (200) superimposed with ablation tags (205, 212); and
a processor (56), which is configured to:
define a nonconductive zone (209) around each ablation tag (205, 212) for at least some of the ablation tags (205, 212), to calculate block areas (214);
receive from a user, markings on the map (200) of a starting point (206A) and an ending point (206B) of a potential conduction flow;
using a search algorithm, search for one or more possible conduction flow paths (210) from the starting point (206A) to the ending point (206B), the one or more existing paths (210) taking into consideration the block areas (214);
overlay one or more flow paths (210), found by the search algorithm, on the anatomical map; and
present the overlayed anatomical map (201) to a user.

9. The system according to claim 8, and comprising not displaying the ablation tags (205, 212) on the overlayed anatomical map (201).

10. The system according to claim 8, wherein the nonconductive zone (209) around a given ablation tag (205, 212) is a sphere of a given size derived from an ablation index value of the given ablation tag.

11. The system according to claim 10, wherein the processor (56) is configured to calculate the block areas (214) by calculating an intersection of the sphere and a surface of the anatomical map.

12. The system according to claim 8, wherein the processor (56) is configured to search for the one or more conduction flow paths (210) by using a first search algorithm.

13. The system according to claim 12, wherein the processor (56) is configured to use a first search algorithm by using an A* path search algorithm.

14. The system according to claim 8, and the processor (56) is further configured to provide a graphical user interface (GUI) configured to allow the user to select at least one of (i) whether to show the ablation tags (205, 212) on the overlayed anatomical map (200), and (ii) whether to calculate sizes of nonconductive zones (209) according to a level of existing ablation therein.

15. The method according to claim 1 or the system according to claim 8, wherein the anatomical map (200) is an electroanatomical (EA) map.

## Patentansprüche

1. Verfahren, umfassend:
Empfangen einer anatomischen Karte (200) von Wandgewebe mindestens eines Abschnitts einer Herzkammer, wobei die Karte mit Ablationsmarkierungen (205, 212) überlagert ist;
Definieren einer nichtleitfähigen Zone (209) um jede Ablationsmarkierung (205, 212) herum für mindestens einige der Ablationsmarkierungen, um Blockbereiche (214) zu berechnen;
Empfangen, von einem Benutzer, von Markierungen auf der Karte (200) eines Startpunkts (206A) und eines Endpunkts (206B) eines potenziellen Leitungsflusses;
unter Verwendung eines Suchalgorithmus, Suchen nach einem oder mehreren möglichen Leitungsflusspfaden (210) von dem Startpunkt (206A) zu dem Endpunkt (206B), wobei der eine oder die mehreren vorhandenen Pfade (210) die Blockbereiche (214) berücksichtigen;
Überdecken eines oder mehrerer Flusspfade (210), die durch den Suchalgorithmus gefunden werden, auf der anatomischen Karte; und
Präsentieren der überdeckten anatomischen Karte (201) an einen Benutzer.

2. Verfahren nach Anspruch 1, und umfassend ein Nichtanzeigen der Ablationsmarkierungen (205, 212) auf der überdeckten anatomischen Karte (201).

3. Verfahren nach Anspruch 1, wobei die nichtleitfähige Zone (209) um eine gegebene Ablationsmarkierung (205, 212) herum eine Kugel einer gegebenen Größe ist, die aus einem Ablationsindexwert der gegebenen Ablationsmarkierung abgeleitet ist.

4. Verfahren nach Anspruch 3, wobei das Berechnen der Blockbereiche (214) das Berechnen eines Schnitts der Kugel und einer Oberfläche der anatomischen Karte (201) umfasst.

5. Verfahren nach Anspruch 1, wobei das Suchen nach dem einen oder den mehreren Leitungsflusspfaden (210) die Verwendung eines ersten Suchalgorithmus umfasst.

6. Verfahren nach Anspruch 5, wobei das Verwenden eines ersten Suchalgorithmus das Verwenden eines A*-Pfadsuchalgorithmus umfasst.

7. Verfahren nach Anspruch 1, und umfassend ein Bereitstellen einer grafischen Bedienerschnittstelle (GUI), die konfiguriert ist, um dem Benutzer zu ermöglichen, mindestens eines auszuwählen von (i) ob die Ablationsmarkierungen (205, 212) auf der überdeckten anatomischen Karte (201) gezeigt werden sollen, und (ii) ob Größen von nichtleitfähigen Zonen (209) gemäß einem Grad an darin vorhandener Ablation berechnet werden sollen.

8. System, umfassend:
eine Schnittstelle (30), die konfiguriert ist, um eine anatomische Karte (200) von Wandgewebe von mindestens einem Abschnitt einer Herzkammer zu empfangen, wobei die Karte (200) mit Ablationsmarkierungen (205, 212) überlagert ist; und
einen Prozessor (56), der konfiguriert ist zum:
Definieren einer nichtleitfähigen Zone (209) um jede Ablationsmarkierung (205, 212) herum für mindestens einige der Ablationsmarkierungen (205, 212), um Blockbereiche (214) zu berechnen;
Empfangen, von einem Benutzer, von Markierungen auf der Karte (200) eines Startpunkts (206A) und eines Endpunkts (206B) eines potenziellen Leitungsflusses;
unter Verwendung eines Suchalgorithmus, Suchen nach einem oder mehreren möglichen Leitungsflusspfaden (210) von dem Startpunkt (206A) zu dem Endpunkt (206B), wobei der eine oder die mehreren existierenden Pfade (210) die Blockbereiche (214) berücksichtigen;
Überdecken eines oder mehrerer Flusspfade (210), die durch den Suchalgorithmus gefunden werden, auf der anatomischen Karte; und
Präsentieren der überdeckten anatomischen Karte (201) an einen Benutzer.

9. System nach Anspruch 8, und umfassend das Nichtanzeigen der Ablationsmarkierungen (205, 212) auf der überdeckten anatomischen Karte (201).

10. System nach Anspruch 8, wobei die nichtleitfähige Zone (209) um eine gegebene Ablationsmarkierung (205, 212) herum eine Kugel einer gegebenen Größe ist, die von einem Ablationsindexwert der gegebenen Ablationsmarkierung abgeleitet ist.

11. System nach Anspruch 10, wobei der Prozessor (56) konfiguriert ist, um die Blockbereiche (214) durch das Berechnen eines Schnitts der Kugel und einer Oberfläche der anatomischen Karte zu berechnen.

12. System nach Anspruch 8, wobei der Prozessor (56) konfiguriert ist, um unter Verwendung eines ersten Suchalgorithmus nach dem einen oder den mehreren Leitungsflusspfaden (210) zu suchen.

13. System nach Anspruch 12, wobei der Prozessor (56) konfiguriert ist, um einen ersten Suchalgorithmus unter Verwendung eines A*-Pfadsuchalgorithmus zu verwenden.

14. System nach Anspruch 8, und der Prozessor (56) ist ferner konfiguriert, um eine grafische Bedienerschnittstelle (GUI) bereitzustellen, die konfiguriert ist, um dem Benutzer zu ermöglichen, mindestens eines auszuwählen von (i) ob die Ablationsmarkierungen (205, 212) auf der überdeckten anatomischen Karte (200) gezeigt werden sollen, und (ii) ob Größen von nichtleitfähigen Zonen (209) gemäß einem Grad an darin vorhandener Ablation berechnet werden sollen.

15. Verfahren nach Anspruch 1 oder System nach Anspruch 8, wobei die anatomische Karte (200) eine elektroanatomische Karte (EA-Karte) ist.

## Revendications

1. Procédé, comprenant :
la réception d'une carte anatomique (200) du tissu de la paroi d'au moins une partie d'une chambre cardiaque, la carte étant superposée à des étiquettes d'ablation (205, 212) ;
la définition d'une zone non conductrice (209) autour de chaque étiquette d'ablation (205, 212) pour au moins certaines des étiquettes d'ablation, pour calculer les zones de bloc (214) ;
la réception, depuis un utilisateur, de marquages sur la carte (200) d'un point de départ (206A) et d'un point d'arrivée (206B) d'un flux de conduction potentiel ;
à l'aide d'un algorithme de recherche, la recherche d'une ou plusieurs voies d'écoulement de conduction (210) possibles entre le point de départ (206A) et le point d'arrivée (206B), la ou les voies existantes (210) prenant en considération les zones de blocs (214) ;
la superposition d'une ou plusieurs voies d'écoulement (210), trouvées par l'algorithme de recherche, sur la carte anatomique ; et
la présentation de la carte anatomique superposée (201) à un utilisateur.

2. Procédé selon la revendication 1, et comprenant le fait de ne pas afficher les étiquettes d'ablation (205, 212) sur la carte anatomique superposée (201).

3. Procédé selon la revendication 1, dans lequel la zone non conductrice (209) autour d'une étiquette d'ablation donnée (205, 212) est une sphère d'une taille donnée dérivée d'une valeur d'indice d'ablation de l'étiquette d'ablation donnée.

4. Procédé selon la revendication 3, dans lequel le calcul des zones de bloc (214) comprend le calcul d'une intersection de la sphère et d'une surface de la carte anatomique (201).

5. Procédé selon la revendication 1, dans lequel la recherche de la ou des voies d'écoulement de conduction (210) comprend l'utilisation d'un premier algorithme de recherche.

6. Procédé selon la revendication 5, dans lequel l'utilisation d'un premier algorithme de recherche comprend l'utilisation d'un algorithme de recherche de chemin A*.

7. Procédé selon la revendication 1, et comprenant la fourniture d'une interface utilisateur graphique (GUI) configurée pour permettre à l'utilisateur de sélectionner au moins l'un parmi (i) l'affichage ou non des étiquettes d'ablation (205, 212) sur la carte anatomique superposée (201), et (ii) le fait de calculer des tailles de zones non conductrices (209) en fonction d'un niveau d'ablation existant dans celles-ci.

8. Système, comprenant :
une interface (30) configurée pour recevoir une carte anatomique (200) du tissu de la paroi d'au moins une partie d'une chambre cardiaque, la carte (200) étant superposée avec des étiquettes d'ablation (205, 212) ; et
un processeur (56), qui est configuré pour :
définir une zone non conductrice (209) autour de chaque étiquette d'ablation (205, 212) pour au moins certaines des étiquettes d'ablation (205, 212), pour calculer les zones de bloc (214) ;
recevoir, depuis un utilisateur, des marquages sur la carte (200) d'un point de départ (206A) et d'un point d'arrivée (206B) d'un flux de conduction potentiel ;
à l'aide d'un algorithme de recherche, rechercher une ou plusieurs voies d'écoulement de conduction (210) possibles du point de départ (206A) au point d'arrivée (206B), la ou les voies existantes (210) tenant compte des zones de bloc (214) ;
superposer une ou plusieurs voies d'écoulement (210), trouvées par l'algorithme de recherche, sur la carte anatomique ; et
présenter la carte anatomique superposée (201) à un utilisateur.

9. Système selon la revendication 8, et comprenant le non-affichage des étiquettes d'ablation (205, 212) sur la carte anatomique superposée (201).

10. Système selon la revendication 8, dans lequel la zone non conductrice (209) autour d'une étiquette d'ablation donnée (205, 212) est une sphère d'une taille donnée dérivée d'une valeur d'indice d'ablation de l'étiquette d'ablation donnée.

11. Système selon la revendication 10, dans lequel le processeur (56) est configuré pour calculer les zones de bloc (214) en calculant une intersection de la sphère et d'une surface de la carte anatomique.

12. Système selon la revendication 8, dans lequel le processeur (56) est configuré pour rechercher la ou les voies d'écoulement de conduction (210) à l'aide d'un premier algorithme de recherche.

13. Système selon la revendication 12, dans lequel le processeur (56) est configuré pour utiliser un premier algorithme de recherche à l'aide d'un algorithme de recherche de chemin A*.

14. Système selon la revendication 8, et le processeur (56) est en outre configuré pour fournir une interface utilisateur graphique (GUI) configurée pour permettre à l'utilisateur de sélectionner au moins l'un parmi (i) l'affichage ou non des étiquettes d'ablation (205, 212) sur la carte anatomique superposée (200), et (ii) l'opportunité de calculer des tailles de zones non conductrices (209) selon un niveau de ablation existante à l'intérieur de celle-ci.

15. Procédé selon la revendication 1 ou système selon la revendication 8, dans lequel la carte anatomique (200) est une carte électroanatomique (EA).
